# EUROPEAN PATENT SPECIFICATION

(11) **EP 1 864 645 B1**
(45) Date of publication and mention of the grant of the patent: **22.05.2013**
(21) Application number: 06730637.3
(22) Date of filing: 30.03.2006
(51) Int. Cl.: A61K 6/083

(54) **FLUORINE ION-RELEASABLE COMPOSITION FOR DENTAL PURPOSES**
FLUORIONEN FREISETZENDE ZUSAMMENSETZUNG FÜR DENTALZWECKE
COMPOSITION LIBERANT DES IONS FLUOR A DES FINS DENTAIRES

(30) Priority: 30.03.2005 JP 2005097353
(43) Date of publication of application: 12.12.2007
(73) Proprietor: Kuraray Noritake Dental Inc., Kurashiki-shi, Okayama 710-0801 (JP)
(72) Inventor: TAKEI, Mitsuru, Kurashiki-shi Okayama 7108622 (JP); KITA, Hirotaka, Kurashiki-shi Okayama 7108622 (JP); OHARA, Yasujiro, Tokyo 1008115 (JP)
(74) Representative: Müller-Boré & Partner Patentanwälte
(86) International application number: PCT/JP2006/306689
(87) International publication number: WO 2006/106838

(56) References cited:
- EP-A- 0 821 931
- EP-A2- 1 101 484
- JP-A- 05 085 915
- JP-A- 10 036 116
- JP-A- 11 158 021
- JP-A- 52 036 892
- NAKABO S. ET AL.: 'Effects of Polysiloxane Coating of NaF on the Release Profile of Fluoride Ion From Bis-GMA/TEGDMA Resin Containing NaF' DENT. MATER. J. vol. 20, no. 1, 2001, pages 53 - 62, XP003003309

## Description

### Technical Field

The present invention relates to a fluoride ion releasing dental composition that exhibits a dental caries preventing effect through a dentine reinforcing function of fluoride ions. Since the fluoride ion releasing dental composition of this invention exhibits the dental caries preventing effect over a long period of time, it is particularly useful as a dental material such as a dental adhesive material, a dental filling material, a dental resin used for forming an abutment, a pit and fissure sealant, a dental coating material (manicure or the like) or a dental cement.

### Background Art

It is well known that fluoride ions work on and reinforce dentine, and a dental material including a fluoride ion releasing material has been already practically used for preventing/suppressing dental caries.

For example, JP 57-88106 describes a dental material including, as a fluoride ion sustained-release material, a homopolymer of fluoride (meth)acrylate or a copolymer of fluoride (meth)acrylate and (meth)acrylic acid lower alkyl ester, and JP 7-101819 describes a dental resin composition including a specific fluorine-containing phosphazene monomer as a fluoride ion sustained-release material.

Furthermore, JP 10-36116 describes a dental composition including an inorganic powder made of composite particles obtained by coating surfaces of substrate particles of a metal fluoride with polysiloxane. In this dental composition, degradation of mechanical properties and adhesion performance caused when a large amount of metal fluoride is included as a fluoride ion releasing material are suppressed through microencapsulation by coating the metal fluoride with polysiloxane.

Document EP-A-0 821 931 discloses a fluoride ion releasing dental composition in which the polysiloxane is coated on the fluoride material in order to control the speed of releasing fluoride ions. Other fillers can be present but not are coated with a polysiloxane.

Moreover, each of Non-patent Documents 1 and 2 specified below describes a pH-responsive fluoride ion sustained-release resin including, as a fluoride ion sustained-release material, a powder made of composite particles obtained by coating surfaces of fluoride particles with a copolymer of tertiary amine having a polymeric group and a monomer. In such a resin, when dental caries is caused to lower the ambient pH, the moisture content of the copolymer used for coating the fluoride is increased so as to rapidly release fluoride ions.

Non-patent Document 1: Dental Materials and Devices, Vol. 22, No. 5 (p. 337)

Non-patent Document 2: Dental Materials and Devises, Vol. 23, No. 5 (p. 443)

### Disclosure of Invention

### Problems to be Solved by Invention

The dental material described in Patent Document 1 and the dental resin composition described in Patent Document 2 have a problem that they cannot provide dentine with high caries resistibility over a long period of time because fluoride ions are completely released in a short period of time of several months in a humid environment of an oral cavity.

The dental composition described in Patent Document 3 also has a problem that it cannot provide dentine with a high cariostatic property over a long period of time because the speed of releasing fluoride ions is increased through the microencapsulation, the reason for which is unknown.

The fluoride sustained-release resins described in Non-patent Documents 1 and 2 have a problem derived from the pH-responsibility that an effect to prevent spread of dental caries, namely, secondary dental caries, can be expected but an effect to prevent primary dental caries can be minimally expected.

Conventionally, a fluoride ion releasing material itself has been improved and an attempt has been made for controlling the speed of releasing fluoride ions by coating particle surfaces of the fluoride ion releasing material with polysiloxane as described above. The fact is, however, that a fluoride ion releasing dental composition capable of providing dentine with a high cariostatic property over a long period of time has not been obtained.

The present invention was devised to overcome the aforementioned problems of the conventional techniques, and an object of the invention is providing a fluoride ion releasing dental composition capable of providing dentine with a high cariostatic property over a long period of time by gradually releasing fluoride ions.

In order to achieve the object, the fluoride ion releasing dental composition according to Item 1 includes an inorganic powder (a); a fluoride ion releasing material (b); a monomer (c); and a polymerization initiator (d), and the inorganic powder (a) is made of composite particles in which a layer of polysiloxane obtained by dehydration condensing a silanol compound obtained through hydrolysis or partial hydrolysis of a silane compound represented by the following Chemical Formula 1 is formed on surfaces of substrate particles made of an inorganic material selected from the group consisting of silicon dioxide, alumina, ceramics, diatomite, kaoline, clay mineral, activated clay, synthesized zeolite, mica, calcium phosphate, barium sulfate, titanium oxide and zirconium oxide, and the fluoride ion releasing material (b) is at least one fluoride ion releasing material selected from the group consisting of ammonium fluoride, ammonium hydrogen fluoride, a metal fluoride, a polymer including, as a constitutional unit, a vinyl monomer having an acid fluoride group and a fluorine-containing phosphazene monomer:

Chemical Formula 1: [(R¹O)₁(X)ₘ]₄₋ₙ-Si(-R²)ₙ

[wherein R¹ is a univalent organic group with a carbon number of 1 through 8, X is a halogen atom, R² is a univalent organic group with a carbon number of 1 through 6, one of 1 and m is 0 and the other is 1, and n is 0 or 1.]

In the fluoride ion releasing dental composition according to Item 2, the inorganic material of the fluoride ion releasing dental composition of Claim 1 is titanium oxide.

In the fluoride ion releasing dental composition according to Item 3, a weight ratio between the fluoride ion releasing material (b) and the polysiloxane of the fluoride ion releasing dental composition is 100:0.1 through 100:1000:

In the fluoride ion releasing dental composition according to Item 4, a weight ratio between the inorganic material and the polysiloxane of the fluoride ion releasing dental composition is 100:20 through 100:2000.

The fluoride ion releasing dental composition of this invention provides dentine with a high cariostatic property over a long period of time by gradually releasing fluoride ions. Although the reason why the fluoride ion releasing dental composition of this invention has a high fluoride ion sustained-release property can be merely presumed, the present inventors presume that it is because fluoride ions freed in a cured substance are once trapped by polysiloxane and then gradually released from the cured substance.

The fluoride ion releasing dental composition of this invention includes an inorganic powder (a), a fluoride ion releasing material (b), a monomer (c) and a polymerization initiator (d).

The inorganic powder (a) is a composite particle powder in which a layer of polysiloxane is formed on surfaces of substrate particles of an inorganic material, and reduces the speed of releasing fluoride ions freed from the fluoride ion releasing material (b) to the outside of a cured substance. Specifically, the polysiloxane present on the surfaces of the particles of the inorganic powder (a) increases the sustained-release property for fluoride ions.

As the inorganic material included in the substrate particles, silicon dioxide (such as quartz, glass or highly dispersible silica), alumina, ceramics, diatomite, kaoline, clay mineral such as montomorillonite, activated clay, synthesized zeolite, mica, calcium phosphate, barium sulfate, titanium oxide and zirconium oxide can be used. In particular, titanium oxide that can function also as a color tone adjuster for a dental material is preferred.

The shape of the substrate particle is not particularly specified and may be any of arbitrary shapes including a spherical shape, a needle shape, a fiber shape and a plate shape. The particle size of the substrate particle is preferably 0.01 through 500 µm and more preferably 0.01 through 50 µm from the viewpoint of handling property.

The polysiloxane formed on the substrate particle in the form of a layer is a polymer having a molecular structure including siloxane bonds (Si-O) linked with one another in the form of a three-dimensional network, and may be organo polysiloxane in which a bivalent organic group is substituted for a part of oxygen atoms bonded to silicon atoms. The polysiloxane can be obtained by a general method, namely, by obtaining a silanol compound through hydrolysis or partial hydrolysis (hereinafter, both of which are expressed as "(partial) hydrolysis") of a hydrolytic silyl group included in a monomer and by dehydration condensing the silanol compound. The inorganic powder (a) can be produced by the following method (1) or (2):
(1) Method in which a silanol compound layer is formed on substrate particles of an inorganic material and silanol groups are intermolecularly dehydration condensed:
   First, a hydrolytic silane compound and a necessary amount of water are added to an organic solvent miscible with water, such as methanol, ethanol or t-butanol, so as to prepare an organic solution including a silanol compound (a hydrolyzed product) through the (partial) hydrolysis of the hydrolytic silane compound in the presence of an acid catalyst. The organic solvent including the silanol compound can be prepared also by preparing an aqueous solution including a silanol compound through (partial) hydrolysis of a hydrolytic silane compound with excessive water added and in the presence of an acid catalyst and subsequently by extracting the silanol compound included in the aqueous solution with an organic solvent (an extract) not miscible with water, such as ethyl acetate, ethyl ether, chloroform or methylene chloride. Then, after adding an inorganic material in the form of a powder to the organic solvent obtained in the aforementioned manner, the organic solvent is vaporized through a heat treatment or a vacuum treatment, so as to give an inorganic powder including composite particles in which the silanol compound is formed in the shape of a layer on a surface of an inorganic material (substrate particles). Subsequently, after adding an acid or base if necessary, the inorganic powder is heated so as to intermolecularly dehydration condense silanol groups, and thus, the inorganic powder (a) including the composite particles in which a polysiloxane layer is formed on the surface of the inorganic material (substrate particles) can be produced. It can be confirmed by using infrared absorption spectra that the layer is made of polysiloxane.
(2) Method in which a hydrolytic silane compound is previously changed into an oligomer by intermolecularly dehydration condensing silanol groups through (partial) hydrolysis, a powdery inorganic material is added to the siloxane oligomer for forming a layer of the siloxane oligomer on a surface of an inorganic material (substrate particles), and the siloxane oligomer is polymerized through intermolecular dehydration condensation:
   First, a given amount of water is added to a hydrolytic silane compound for performing (partial) hydrolysis in the presence of an acid catalyst while distilling the resultant solution to take away alcohol generated as a by-product, so as to produce a siloxane oligomer. A powdery inorganic material is added to the siloxane oligomer so as to form a layer of the siloxane oligomer on the surfaces of particles, and after adding an acid or base if necessary, silanol groups included in the siloxane oligomer are intermolecularly dehydration condensed through a heat treatment. Thus, the inorganic powder (a) including composite particles in which a polysiloxane layer is formed on the surfaces of substrate particles made of the inorganic material is produced.

The hydrolytic silane compound used as a starting material for the polysiloxane is a silane compound represented by the following Chemical Formula 2, in which silanol groups are generated through the hydrolysis and the thus generated silanol groups are intermolecularly dehydration condensed so as to produce polysiloxane:

Chemical Formula 2: [(R¹O)₁(X)ₘ]₄₋ₙ-Si (-R²)ₙ

[wherein R¹ is a univalent organic group with a carbon number of 1 through 8, X is a halogen atom, R² is a univalent organic group with a carbon number of 1 through 6, one of 1 and m is 0 and the other is 1, and n is 0 or 1.]

In Chemical Formula 2, each of the R¹O group and the X group is a functional group or an atom generating a silanol group through hydrolysis. Examples of the R¹ group are methyl, ethyl, 2-chloroethyl, allyl, aminoethyl, propyl, isopentyl, hexyl, 2-methoxyethyl, phenyl, m-nitrophenyl and 2,4-dichlorophenyl, and in particular, methyl and ethyl are preferred. Examples of the X group are chlorine and bromine, and in particular, chlorine is preferred. Examples of the R² group are methyl, chloromethyl, bromoethyl, ethyl, vinyl, 1,2-dibromovinyl, 1,2-dichloroethyl, 2-cyanoethyl, diethylaminoethyl, 2-aminoethylaminoethyl, 2-(2-aminoethyl thioethoxy), propyl, isopropyl, 3-hydroxypropyl, 3-mercaptopropyl, 3-aminopropyl, 3,3,3-trifluoropropyl, 3-piperazinopropyl, 3-glycidoxypropyl, 3-(2-aminoethylamino)propyl, allyl, n-butyl, isobutyl, hexyl, cyclohexyl, 3-methacryloyloxypropyl and phenyl, and in particular, methyl, ethyl, propyl, vinyl, 3-methacryloyloxypropyl and phenyl are preferred.

In Chemical Formula 2, examples of the silane compound in which n = 0 are tetramethoxysilane, tetraethoxysilane, tetraallyloxysilane, tetrabutoxysilane, tetra(2-ethylhexyloxy)silane, diethoxydichlorosilane, tetraphenoxysilane and tetrachlorosilane, and in particular, tetramethoxysilane and tetraethoxysilane are preferred.

In Chemical Formula 2, examples of the silane compound in which n = 1 are methyltrimethoxysilane, ethyltriethoxysilane, propyltriethoxysilane, hexyltrimethoxysilane, vinyltriethoxysilane, 3-methachloyloxypropyltrimethoxysilane, vinyltriethoxysilane, 3-aminopropylethoxysilane, methyltrichlorosilane and phenyltrichlorosilane, and in particular, methyltrimethoxysilane, ethyltriethoxysilane, vinyltriethoxysilane, 3-methacryloyloxypropyltrimethoxysilane and phenyltrichlorosilane are preferred.

One of the aforementioned silane compounds may be singly used or a plurality of them may be used together.

The weight ratio between the inorganic material (substrate particles) and the polysiloxane (surface layer) in the inorganic powder (a) is preferably 100:20 through 100:200, more preferably 100:50 through 100:1500 and most preferably 100:100 through 100:1000. In the case where the ratio of the polysiloxane to the inorganic material is excessively small, the fluoride ion sustained-release property may not be sufficiently improved, and on the other hand, in the case where the ratio is excessively large, the surface layer is so thick that the handling property of the composition as a dental material may be lowered.

The content of the inorganic powder (a) is preferably 0.1 through 60 wt%, more preferably 0.5 through 40 wt% and most preferably 1 through 20 wt% based on the total weight of the composition. In the case where the content is smaller than 0.1 wt%, it is apprehended that the fluoride ion sustained-release property cannot be sufficiently improved, and on the other hand, in the case where the content exceeds 60 wt%, the handling property of the composition as a dental material may be lowered.

In order to suppress precipitation of the inorganic powder (a) or to improve the mechanical strength of a cured substance and the coating property, easiness in taking out of a container, operability and the like of the composition, another powder may be used together with the inorganic powder (a). Examples of another powder to be used with the inorganic powder (a) are an inorganic filler, an organic filler and an inorganic/organic complex filler.

Examples of the inorganic filler are the inorganic materials described as the examples of the inorganic material used as the substrate particles of the inorganic powder (a).

Examples of the organic filler are polymethyl methacrylate, polyethyl methacrylate, a polymer of polyfunctional methacrylate, polyamide, polystyrene, polyvinyl chloride, chloroprene rubber, nitrile rubber and styrene-butadiene rubber.

Examples of the inorganic/organic complex filler are a filler obtained by dispersing an inorganic filler in an organic matrix and an inorganic filler obtained by coating particles with any of various monomers and curing it.

Another powder to be used together with the inorganic powder (a) may be previously subjected to a surface treatment with a known surface-treatment agent such as a silane coupling agent. Examples of the surface-treatment agent are vinyltrimethoxysilane, vinyltriethoxysilane, vinyltrichlorosilane, vinyltri(ß-methoxyethoxy)silane, γ-methacryloyloxypropyltrimethoxysilane, γ-glycidoxypropyltrimethoxysilane, γ-mercaptopropyltrimethoxysilane and γ-aminopropyltriethoxysilane.

The average particle diameter of another powder to be used together with the inorganic powder (a) is preferably 0.001 through 50 µm and more preferably 0.001 through 10 pm, and in general, the content is preferably 0.1 through 60 wt% and more preferably 0.1 through 40 wt% based on the total weight of the composition. In the case where the content exceeds 60 wt%, the handling property of the composition may be lowered.

Fluoride ions released from the fluoride ion releasing material (b) work on dentine for reinforcing it. The fluoride ion releasing material (b) is at least one fluoride ion releasing material selected from the group consisting of ammonium fluoride, ammonium hydrogen fluoride, a metal fluoride, a polymer including, as a constitutional unit, a vinyl monomer having an acid fluoride group and a fluorine-containing phosphazene monomer. The polymer including, as a constitutional unit, a vinyl monomer having an acid fluoride group and the fluorine-containing phosphazene monomer may be soluble or insoluble in the monomer (c).

Examples of the polymer including, as a constitutional unit, a vinyl monomer having an acid fluoride group are a homopolymer of fluoride (meth)acrylate represented by the following Chemical Formula 3, and a copolymer of this fluoride (meth)acrylate and alkyl (meth)acrylate represented by the following Chemical Formula 4 (such as methyl (meth)acrylate, ethyl (meth)acrylate, butyl (meth)acrylate and octyl (meth)acrylate) (see Japanese Laid-Open Patent Publication No. Sho 62-12706): 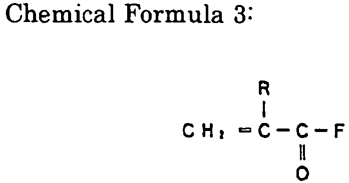 [wherein R is H or CH₃.] 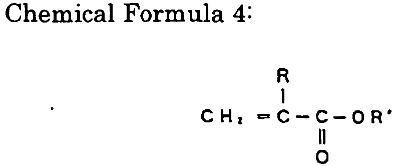 [wherein R is H or CH₃ and R' is an alkyl group with a carbon number of 1 through 8.]

The homopolymer and the copolymer can be produced through a radical polymerization reaction. Specifically, in the presence or absence of a solvent, a heat treatment is performed for polymerization with an appropriate initiator added. The polymerization reaction is controlled and a product is obtained in accordance with a technique established with respect to production of a polymer of a derivative of (meth)acrylic acid. Such a polymer gradually releases fluoride ions in water probably because a -C(O)-F portion of the polymer is gradually hydrolyzed to be changed into -C(O)-OH and HF is freed. The total amount of fluoride ions released from the polymer is larger as the proportion of a unit including the -C(O)-F portion is larger, namely, as the proportion of the fluoride (meth)acrylate in the polymer is larger, and the releasing speed is higher also as the proportion of the fluoride (meth)acrylate is larger. In order to obtain a material that releases fluoride ions in an amount for effectively fluoridating dentine, a homopolymer of fluoride (meth)acrylate or a copolymer of fluoride (meth)acrylate and (meth)acrylic acid alkyl ester in which the proportion of fluoride (meth)acrylate is 10 mol% or more (more preferably 20 through 80 mol%) is preferably used. In particular, such a polymer in which the copolymerization amount of fluoride (meth)acrylate is 20 through 80 mol% is practically used because it can secure a sustained-release amount of fluorine and is well dissolved in (meth)acrylic acid ester monomer.

An example of the fluorine-containing phosphazene monomer is a cyclic phosphazene compound including a constitutional unit represented by the following Chemical Formula 5 (see Japanese Laid-Open Patent Publication No. Hei 7-101819): 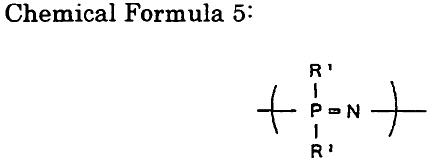 [wherein at least one of R¹ and R² is F, and the rest are the same as or different from each other and have a polymeric double bond.]

Examples of the cyclic phosphazene compound are a 6-membered cyclic compound and an 8-membered cyclic compound. An example of the group having a polymeric double bond is a (meth)acryloyloxyalkyl group such as a 2-(meth)acryloyloxymethoxy group, a 2-(meth)acryloyloxyethoxy group, a 2-(meth)acryloyloxypropoxy group or a 2-(meth)acryloyloxybutoxy group. Specific examples of the cyclic phosphazene compound are a 6-membered cyclic compound represented by P₃N₃(F)ₙ[O(CH₂)₂COO(CH₃)C=CH₂]₆₋ₙ (wherein n is an integer of 1 through 5) and an 8-membered cyclic compound represented by P₄N₄(F)ₙ[(CH₂)₂COO(CH₃)C=CH₂]₈₋ₘ (wherein m is an integer of 1 through 7). Such a cyclic phosphazene compound can be obtained by allowing, for example, a 6-membered cyclic compound of P₃N₃F₆ or an 8-membered cyclic compound of P₄N₄F₈ to react with hydroxyethyl methacrylate.

One of the aforementioned organic fluoride ion releasing materials may be singly used or a plurality of them may be used together. It is noted that fluorine-containing glass such as fluoroaluminosilicate, aluminoborate, borosilicate or boroaluminosilicate is not used as the fluoride ion releasing material of this invention because such a material releases a small amount of fluoride ions and has a small effect to improve the cariostatic property of dentine.

One of ammonium fluoride, ammonium hydrogen fluoride and a metal fluoride may be singly used or a plurality of them may be used together. As such an inorganic fluoride ion releasing material, any of fluoride ion releasing materials known as dental materials may be used. The shape of the inorganic fluoride ion releasing material is not particularly specified, and may be any of arbitrary shapes including a spherical shape, a needle shape, a plate shape, a crushed shape and a scaly shape. The average particle diameter of the inorganic fluoride ion releasing material is preferably 0.01 through 30 µm and more preferably 0.01 through 10 µm. One of inorganic fluoride ion releasing materials may be singly used or a plurality of them may be used together.

Examples of the metal fluoride are lithium fluoride, sodium fluoride, potassium fluoride, rubidium fluoride, cesium fluoride, beryllium fluoride, magnesium fluoride, calcium fluoride, strontium fluoride, barium fluoride, aluminum fluoride, manganese (II) fluoride, iron (II) fluoride, iron (III) fluoride, cobalt (II) fluoride, copper (II) fluoride, zinc fluoride, antimony (III) fluoride, lead (II) fluoride, silver (I) fluoride, cadmium fluoride, tin (II) fluoride, tin (IV) fluoride, diamine silver fluoride, sodium hydrogen fluoride, potassium hydrogen fluoride, sodium fluorophosphate, potassium hexafluorotitanate, sodium hexafluorosilicate, sodium hexafluorophosphate, sodium hexafluorostannate (IV), alanine hexafluorostannate (IV), sodium pentafluorodistannate (II) and potassium hexafluorozirconate.

Among the exemplified metal fluorides, a fluoride of a metal of group I or II of the periodic table, such as lithium fluoride, sodium fluoride, potassium fluoride, rubidium fluoride, cesium fluoride, beryllium fluoride, magnesium fluoride, calcium fluoride, strontium fluoride or barium fluoride, is preferred and sodium fluoride is most preferred. One of these metal fluorides may be singly used or a plurality of them may be used together.

The inorganic fluoride ion releasing material may be subjected to a surface treatment with an organic chelate compound, an acidic group-containing compound or the like. Alternatively, a complex of an inorganic fluoride ion releasing material and an organic material such as a monomer may be used. The content of the fluoride ion releasing material (b) is preferably 0.01 through 70 wt% and more preferably 0.01 through 60 wt% based on the total weight of the composition. In the case where the content is smaller than 0.01 wt%, fluoride ions may not be released in a sufficient amount, and on the other hand, in the case where the content exceeds 70 wt%, the handling property of the composition as a dental material may be lowered.

The monomer (c) is appropriately selected in accordance with the purpose/use of the composition in the dental care. Examples of the monomer (c) are esters of unsaturated organic acids such as α-cyanoacrylate, (meth)acrylate, α-halogenated acrylate, crotonate, cinnamate, sorbate, maleate and itaconate, (meth)acrylamide and a derivative thereof, vinyl esters, vinyl ethers, a mono-N-vinyl derivative and a styrene derivative. In particular, (meth)acrylate is preferred. One of these monomers (c) may be singly used or a plurality of them may be used together.

Specific examples of the (meth)acrylate are as follows, in which a monomer having n (wherein n is a natural number) olefin double bonds is expressed as an n-functional monomer:
(A) Monofunctional monomers:
   methyl (meth)acrylate, isobutyl (meth)acrylate, benzyl (meth)acrylate, lauryl (meth)acrylate, 2-hydroxyethyl (meth)acrylate, 2-(N,N-dimethylamino)ethyl (meth)acrylate, 2,3-dibromopropyl (meth)acrylate, oxysilanylmethyl (meth)acrylate, 3-methacryloyloxypropyltrimethoxysilane
(B) Bifunctional monomers:
   ethylene glycol di(meth)acrylate, triethylene glycol di(meth)acrylate, propylene glycol di(meth)acrylate, neopentyl glycol di(meth)acrylate, 1,6-hexanediol di(meth)acrylate, 1,10-decanediol di(meth)acrylate, bisphenol A diglycidyl (meth)acrylate, 2,2-bis[(meth)acryloyloxyethoxyphenyl]propane, 2,2-bis[(meth)acryloyloxypolyethoxyphenyl]propane, 2,2-bis[4-[3-(meth)acryloyloxy-2-hydroxypropoxy]phenyl]propane, 1,2-bis[3-(meth)acryloyloxy-2-hydroxypropoxy]ethane
(C) Tri- or multi functional monomers:
   trimethylolpropane tri(meth)acrylate, trimethylolethane tri(meth)acrylate, tetramethylolmethane tri(meth)acrylate, pentaerythritol tetra(meth)acrylate

The content of the monomer (c) is appropriately set in accordance with the use. From the viewpoint of handling property, the content is preferably 20 through 99 wt% and more preferably 40 through 95 wt% based on the total weight of the composition.

For the purpose of securing high adhesiveness to dentine or a repaired substance, an acidic group-containing monomer may be used as a part of the monomer (c). The acidic group-containing monomer is a monomer having an acidic group such as a phosphate group, a pyrophosphate group, thiophosphate group, a carboxylate group or a sulfonate group and a polymerizable unsaturated group such as an acryloyl group, methacryloyl group, a vinyl group or a vinyl benzyl group.

Specific examples of the monomer including a phosphate group as the acidic group are 2-(meth)acryloyloxyethyl dihydrogenphosphate, 10-(meth)acryloyloxydecyl dihydrogenphosphate, 20-(meth)acryloyloxyicosyl dihydrogenphosphate, [2-(meth)acryloyloxy-1-[(meth)acryloyloxymethyl]ethyl] dihydrogenphosphate, 2-(meth)acryloyloxyethylphenyl phosphate, 2-(2-(meth)acryloyloxyethyl)-2-bromoethyl phosphate, (meth)acryloyloxyethylphenyl phosphonate and acid chlorides thereof.

Specific examples of the monomer including a pyrophosphate group as the acidic group are bis(2-(meth)acryloyloxyethyl) pyrophosphate and an acid chloride thereof.

Specific examples of the monomer including a thiophosphate group as the acidic group are 2-(meth)acryloyloxyethyl dihydrogenthiophosphate, 10-(meth)acryloyloxydecyl dihydrogenthiophosphate and acid chlorides thereof. Specific examples of the monomer including a carboxylate group as the acidic group are 4-(meth)acryloyloxyethoxy carbonylphthalate and an anhydride thereof, 5-(meth)acryloylamino pentanoate, 11-(meth)acryloyloxy-1,1-undecanedicarboxylate and acid chlorides thereof.

Specific examples of the monomer including a sulfonate group as the acidic group are a compound including a sulfonate group such as 2-(meth)acrylamideethyl sulfonic acid, 3-(meth)acrylamidepropyl sulfonic acid, 4-(meth)acrylamidebutyl sulfonic acid, 6-(meth)acrylamidehexyl sulfonic acid, 8-(meth)acrylamideoctyl sulfonic acid, 10-(meth)acrylamidedecyl sulfonic acid or styrene sulfonic acid, and acid chlorides, alkali metal salts and ammonium salts thereof.

One of the acidic group-containing monomers may be singly used or a plurality of them may be used together. The content of the acidic group-containing monomer is preferably not more than 60 wt% and more preferably not more than 40 wt% based on the total weight of the composition. In the case where the content exceeds 60 wt%, the curing property of the composition may be lowered.

As the polymerization initiator (d), any of known photopolymerization initiators and chemical polymerization initiators can be used. A photopolymerization initiator and a chemical polymerization initiator may be used together.

Examples of the photopolymerization initiator are α-diketones, ketals, thioxanthones, acylphosphine oxides and α-aminoacetophenones.

Specific examples of the α-diketones are camphorquinone, benzyl and 2,3-pentanedione.

Specific examples of the ketals are benzyl dimethyl ketal and benzyl diethyl ketal.

Specific examples of the thioxanthones are 2-chlorothioxanthone and 2,4-diethylthioxanthone.

Specific examples of the acylphosphine oxides are 2,4,6-trimethylbenzoyldiphenylphosphine oxide, bis(2,4,6-trimethylbenzoyl)phenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, benzoyl-bis(2,6-dimethylphenyl)phosphine oxide, 2,4,6-trimethylbenzoylethoxyphenylphosphine oxide and a water-soluble acylphosphine oxide compound disclosed in Japanese Laid-Open Patent Publication No. Hei 3-57916.

Specific examples of the α-aminoacetophenones are 2-benzyl-2-dimethylamino-1-(4-morpholinophenyl)-butanone-1, 2-benzyl-2-diethylamino-1-(4-morpholinophenyl)-butanone-1, 2-benzyl-2-dimethylamino-1-(4-morpholinophenyl)-propanone-1, 2-benzyl-2-diethylamino-1-(4-morpholinophenyl)-propanone-1, 2-benzyl-2-dimehylamino-1-(4-morpholinophenyl)-pentanone-1 and 2-benzyl-2-diethylamino-1-(4-morpholinophenyl)-pentanone-1.

One of these photopolymerization initiators may be singly used or a plurality of them may be used together. The content of the photopolymerization initiator is preferably 0.01 through 10 wt%, more preferably 0.05 through 7 wt% and most preferably 0.1 through 5 wt% based on the monomer (c).

In order to improve the photo-setting property, a photopolymerization initiator may be used together with a polymerization promoter such as tertiary amines, aldehydes and thiol compounds.

Specific examples of the tertiary amines are 2-dimethylaminoethyl (meth)acrylate, N,N-bis[(meth)acryloyloxyethyl]-N-methylamine, ethyl 4-dimethylaminobenzoate, butyl 4-dimethylaminobenzoate, butoxyethyl 4-dimethylaminobenzoate, N-methyl diethanolamine and 4-dimethylaminobenzophenone.

Specific examples of the aldehydes are dimethylaminobenzaldehyde and terephthalaldehyde.

Specific examples of the thiol compounds are 2-mercaptobenzooxazole, decanethiol, 3-mercaptopropyltrimethoxysilane and thiobenzoic acid.

One of the polymerization promoters may be singly used or a plurality of them may be used together. The content of the polymerization promoter is preferably 0.01 through 10 wt%, more preferably 0.05 through 7 wt% and most preferably 0.1 through 5 wt% based on the monomer (c).

As the chemical polymerization initiator, a redox polymerization initiator composed of an oxidant and a reductant is preferably used. In the case where a redox chemical polymerization initiator is used, the composition is dividedly packaged into two or more packs because the oxidant and the reductant should be separated from each other until immediately before use.

Examples of the oxidant are organic peroxides such as diacyl peroxides, peroxy esters, dialkyl peroxides, peroxy ketals, ketone peroxides and hydroperoxides.

Specific examples of the diacyl peroxides are benzoyl peroxide, 2,4-dichlorobenzoyl peroxide and m-toluoyl peroxide.

Specific examples of the peroxy esters are t-butyl peroxybenzoate, bis-t-butyl peroxyisophthalate, 2,5-dimethyl-2,5-bis(benzoylperoxy)hexane, t-butyl peroxy-2-ethyl hexanoate and t-butyl peroxyisopropyl carbonate.

Specific examples of the dialkyl peroxides are dicumyl peroxide, di-t-butyl peroxide and lauroyl peroxide.

A specific example of the peroxy ketals is 1,1-bis(t-butyl peroxy)-3, 3, 5-trimethylcyclohexane.

Specific examples of the ketone peroxides are methyl ethyl ketone peroxide, cyclohexanone peroxide and methyl acetoacetate peroxide.

Specific examples of the hydroperoxides are t-butyl hydroperoxide, cumene hydroperoxide and p-diisopropyl benzene peroxide.

As the reductant, aromatic tertiary amine, aliphatic tertiary amine, sulfinic acid and salts thereof are preferably used.

Specific examples of the aromatic tertiary amine are N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N, N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3, 5-diisopropylaniline, N,N-bis(2-hydroxyethyl)-3,5-dibutylaniline, ethyl 4-dimethylaminobenzoate, n-butoxyethyl 4-dimethylaminobenzoate and 2-methacryloyloxyethyl 4-dimethylaminobenzoate.

Specific examples of the aliphatic tertiary amine are trimethylamine, triethylamine, N-methyldiethanolamine, N-ethyldiethanolamine, N-n-butyldiethanolamine, N-lauryldiethanolamine, triethanolamine, 2-dimethylaminoethyl methacrylate, N-methyldiethanolamine dimethacrylate, N-ethyldiethanolamine dimethacrylate, triethanolamine monomethacrylate, triethanolamine dimethacrylate and triethanolamine trimethacrylate.

Specific examples of the sulfinic acid and salts thereof are benzenesulfinic acid, sodium benzenesulfinate, potassium benzenesulfinate, calcium benzenesulfinate, lithium benzenesulfinate, toluenesulfinic acid, sodium toluenesulfinate, potassium toluenesulfinate, calcium toluenesulfinate, lithium toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisorpopylbenzenesulfinic acid, sodium 2,4,6-triisopropylbenzenesulfinate, potassium 2,4,6-triisopropylbenzenesulfinate and calcium 2,4,6-triisopropylbenzenesulfinate.

With respect to each of the oxidant and the reductant, one kind alone may be used or a plurality of kinds may be used together. The content of each of the oxidant and the reductant is preferably 0.01 through 10 wt%, more preferably 0.05 through 7 wt% and most preferably 0.1 through 5 wt% based on the monomer (c).

The fluoride ion releasing dental composition of this invention may include, if necessary, a polymerization inhibitor, a pigment, a coloring agent, a fluorescent agent, a UV absorber, an antibacterial material and so on in amount not spoiling the effects of the invention.

### Embodiments

The present invention will now be described in detail on the basis of preferred embodiments thereof, and it is noted that the invention is not limited to the following embodiments. Abbreviations used in description below stand for the following:

### [Fluoride ion releasing material (b)]

NaF: sodium fluoride
40PMF: polymer represented by the following Chemical Formula 6: 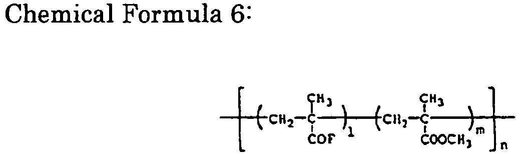 wherein l:m = 2:3 and n = 100.

### [Monomer (c)]

3G: triethylene glycol dimethacrylate
Bis-GMA: bisphenol A diglycidyl methacrylate
HEMA: 2-hydroxyethyl methacrylate
MDP: 10-methacryloyloxydecyl dihydrogenphosphate
[Polymerization initiator (d)]
CQ: camphorquinone
DMABE: ethyl 4-dimethylaminobenzoate (polymerization promoter)

### [Inorganic powder (a)]

Ten kinds of inorganic powders (a1) through (a10) (all of which are inorganic powders according to the present invention) listed in Table 1 were produced by forming a polysiloxane layer on surfaces of particles of titanium oxide or sodium fluoride by methods described below.

### (Production of inorganic powder (a1))

To 34.7 g of tetraethoxysilane, 12 g of water in an equimolar amount to ethoxy groups of the tetraethoxysilane, 10 g of ethanol and 0.02 g of hydrochloric acid were added, and the resultant was heated and refluxed for 2 hours with stirring so as to hydrolyze the tetraethoxysilane. Ten g of a titanium oxide powder was added to the thus obtained solution, and the solution was stirred and distilled to take away ethanol under reduced pressure. Subsequently, a heat treatment was performed at 120°C for 30 minutes, so as to give 19 g of a white inorganic powder (a1). This inorganic powder (a1) was washed with ethyl acetate, and no silane compound was eluted into the wash solution. It was confirmed based on this fact that a hydrolysate of the tetraethoxysilane was condensation polymerized on the particle surfaces of the titanium oxide to be insolubilized. Furthermore, through comparison of infrared absorption spectra of the tetraethoxysilane and infrared absorption spectra of a constitutional substance of a layer formed on the particle surfaces of the titanium oxide, it was found that absorption of an ethoxy group appearing at 960 cm⁻¹ and 1170 cm⁻¹ of the infrared absorption spectra of the tetraethoxysilane disappeared but broad absorption of SiO₂ appeared in the vicinity of 1000 through 1200 cm⁻¹ in the infrared absorption spectra of the constitutional substance. It was confirmed based on this fact that the tetraethoxysilane was hydrolyzed and then dehydration condensed into polysiloxane.

### (Production of inorganic powders (a2) through (a4))

A silane compound (alkoxysilane) listed in Table 1 and titanium oxide were used so as to hydrolyze the silane compound and then to dehydration condense it on particle surfaces of the titanium oxide by a similar method to that employed for the inorganic powder (a1), so as to give each of white inorganic powders (a2) through (a4).

### (Production of inorganic powder (a5))

To 34.7 g of tetraethoxysilane, 12 g of water in an equimolar amount to ethoxy groups of the tetraethoxysilane, 10 g of ethanol and 0.02 g of hydrochloric acid were added, and the resultant was heated and refluxed for 2 hours with stirring so as to hydrolyze the tetraethoxysilane. Ten g of a sodium fluoride powder was added to the thus obtained solution, and the solution was stirred and distilled to take away ethanol under reduced pressure. Subsequently, a heat treatment was performed at 120°C for 30 minutes, so as to give 19 g of a white inorganic powder (a5). This powder was washed with ethyl acetate, and no silane compound was eluted into the wash solution. It was confirmed based on this fact that the tetraethoxysilane was hydrolyzed and then condensation polymerized on the particle surfaces of the sodium fluoride to be insolubilized. Furthermore, through comparison of infrared absorption spectra of the tetraethoxysilane and infrared absorption spectra of the constitutional substance of a layer formed on the particle surfaces of the sodium fluoride, it was found that absorption of an ethoxy group appearing at 960 cm⁻¹ and 1170 cm⁻¹ of the infrared absorption spectra of the tetraethoxysilane disappeared but broad absorption of SiO₂ appeared in the vicinity of 1000 through 1200 cm⁻¹ in the infrared absorption spectra of the constitutional substance. It was confirmed based on this fact that the tetraethoxysilane was hydrolyzed and then dehydration condensed into polysiloxane.

### (Production of inorganic powder (a6))

To a mixed solution of 100 g of vinyltriethoxysilane and 100 g of water, 0.2 g of acetic acid was added, and the resultant solution was stirred at room temperature until the solution became homogeneous. After adding a saturated brine to this aqueous solution, the resultant was extracted with ethyl acetate. The thus obtained ethyl acetate solution was washed with a sodium hydrogencarbonate aqueous solution so as to remove acetic acid, and then, the ethyl acetate solution was dried with anhydrous sodium sulfate and anhydrous magnesium sulfate. The desiccating agents were removed through filtration, and the resultant was distilled to take away the ethyl acetate under reduced pressure, so as to give 23 g of a hydrolysate of the vinyltriethoxysilane. Subsequently, 10 g of the hydrolysate was dissolved in 10 g of toluene, and 0.5 g of 3-aminopropyltriethoxysilane was further added as a curing catalyst. Ten g of a titanium oxide powder was added to the thus obtained solution, and the solution was stirred and distilled to take away toluene under reduced pressure. Subsequently, a heat treatment was performed at 120°C for 30 minutes, so as to give 19 g of a white inorganic powder (a6). This inorganic powder (a6) was washed with toluene, and no silane compound was eluted into the wash solution. It was confirmed based on this fact that the hydrolysate of the vinyltriethoxysilane was condensation polymerized on the particle surfaces of the titanium oxide to be insolubilized. Furthermore, through comparison of infrared absorption spectra of the vinyltriethoxysilane and infrared absorption spectra of a constitutional substance of a layer formed on the particle surfaces of the titanium oxide, it was found that absorption of an ethoxy group appearing at 950 cm⁻¹ and 1170 cm⁻¹ of the infrared absorption spectra of the vinyltriethoxysilane disappeared but broad absorption of SiO₂ appeared in the vicinity of 1000 through 1200 cm⁻¹ in the infrared absorption spectra of the constitutional substance. It was confirmed based on this fact that the vinyltriethoxysilane was hydrolyzed and then dehydration condensed into polysiloxane.

### (Production of inorganic powders (a7) through (a9))

Each silane compound (alkoxysilane) listed in Table 1 and titanium oxide were used so as to hydrolyze the silane compound and to dehydration condense it on particle surfaces of the titanium oxide by a similar method to that employed for the inorganic powder (a1), so as to give each of white inorganic powders (a7) through (a9).

### (Production of inorganic powder (a10))

Ten g of a siloxane oligomer (manufactured by Mitsubishi Chemical Corporation, trade name "MSAC") was dissolved in 10 g of toluene, and 0.1 g of nitric acid was further added thereto as a curing catalyst. Ten g of a titanium oxide powder was added to the thus obtained solution, and the solution was stirred and distilled to take away toluene under reduced pressure. Subsequently, a heat treatment was performed at 120°C for 30 minutes, so as to give 18 g of a white inorganic powder (a10). This inorganic powder (a10) was washed with toluene, and no silane compound was eluted into the wash solution. It was confirmed based on this fact that the siloxane oligomer was crosslinked on the particle surfaces of the titanium oxide to be changed into polysiloxane and to be insolubilized.

**Table 1:**

| Inorganic powder (a) | Silane compound (polysiloxane material) | Inorganic material | Ratio of polysiloxane material per 100 parts by weight of inorganic material (parts bv weight) |
|---|---|---|---|
| Inorganic powder (a1) | Tetraethoxysilane | Titanium oxide | 100 |
| Inorganic powder (a2) | Tetraethoxysilane | Titanium oxide | 300 |
| Inorganic powder (a3) | Tetraethoxvsilane | Titanium oxide | 30 |
| Inorganic powder (a4) | Tetraethoxvsilane | Titanium oxide | 10 |
| Inorganic powder (a5) | Tetraethoxysilane | NaF | 100 |
| Inorganic powder (a6) | Vinylethoxysilane | Titanium oxide | 100 |
| Inorganic powder (a7) | Methyltriethoxvsilane | Titanium oxide | 100 |
| Inorganic powder (a8) | Methyltriethoxvsilane | Titanium oxide | 700 |
| Inorganic powder (a9) | 3-methacryloyloxypropyl trimethoxysilane | Titanium oxide | 30 |
| Inorganic powder (a10) | MSAC (oligomer) | Titanium oxide | 100 |

### (Embodiment 1)

A fluoride ion releasing dental composition was prepared by mixing the inorganic powder (a1) (15 wt%), 40PMF (10 wt%), 3G (45 wt%), Bis-GMA (28.5 wt%), CQ (0.5 wt%) and DMABE (1 wt%). Subsequently, the fluoride ion releasing property of the fluoride ion releasing dental composition was examined by a method described in (1) below, and the acid resistance of enamel attained by applying the fluoride ion releasing dental composition to the enamel was examined by a method described in (2) below. The results are listed in Table 2.

### (1) Fluoride ion releasing property

The fluoride ion releasing dental composition was filled in a mold with a diameter of 20 mm and a thickness of 1 mm and cured by irradiating for 30 seconds with a dental light irradiator (manufactured by Gunma Ushio Electric Inc., trade name "Lightel II"), and the thus obtained cured substance was taken out of the mold and immersed in 10 ml of phosphate buffer (with pH 7 kept at 37°C). Fluoride ions eluted into the phosphate buffer were determined with a fluoride ion electrode 30 days, 60 days, 120 days, 240 days and 360 days after the immersion, so as to calculate an average fluoride ion releasing amount (µg/g/day) per gram of the cured substance and per day attained in each of periods of 1 through 30 days, 31 through 60 days, 61 through 120 days, 121 through 240 days and 241 through 360 days after the immersion. Average fluoride ion releasing amounts mentioned in embodiments and comparative examples described below were obtained in the same manner.

### (2) Acid resistance of enamel

The fluoride ion releasing dental composition was applied to buccal enamel of an evulsed human bicuspid tooth and cured by irradiating for 30 seconds with a dental light irradiator (the above-described "Lightel II"), and then, after immersing the resultant tooth in a phosphate buffer (with pH 7 kept at 37°C) for 360 days, the cured substance was removed so as to expose the enamel. Subsequently, the dentine surface was coated with a dental sealant excluding a circular window portion with a diameter of 3 mm, and the resultant tooth was immersed in an acetate buffer (kept at 37°C) with pH 4.6, which is equal to pH of dental plaque, for 1 week. Thereafter, calcium ions eluted into the acetate buffer from the enamel during the immersion were determined with a calcium ion electrode, so as to calculate an average calcium ion eluting amount (µg/cm²/hr) per area of 1 cm² of the enamel and per hour. Average calcium ion eluting amounts mentioned in the embodiments and comparative examples described below were obtained in the same manner.

### (Embodiments 2 through 7)

Six kinds of fluoride ion releasing dental compositions having compositions listed in Table 2 were prepared, and the average fluoride ion releasing amounts and the average calcium ion eluting amounts were respectively obtained. The results are shown in Table 2. Embodiment 8 is not within the scope of the invention.

### (Comparative Example 1)

A solution was obtained by dissolving 10 g of 3-methacryloyloxypropyltrimethoxysilane in 10 g of toluene, and 10 g of a titanium oxide powder was added thereto and the resultant solution was stirred. Then, the solution was distilled to take away toluene under reduced pressure, and subsequently, a heat treatment was performed at 90°C for 3 hours, so as to give 18 g of silanized titanium oxide. A fluoride ion releasing dental composition having a composition listed in Table 2 was prepared in the same manner as in Embodiment 2 except that this silanized titanium oxide was used instead of the inorganic powder (a1), and the average fluoride releasing amount and the average calcium eluting amount of this fluoride ion releasing dental composition were obtained. The results are shown in Table 2.

### (Comparative Example 2)

A solution was obtained by dissolving 10 g of 3-methacryloyloxypropyltrimethoxysilane in 10 g of toluene, and 10 g of a titanium oxide powder was added thereto and the resultant solution was stirred. Then, the solution was distilled to take away toluene under reduced pressure, and subsequently, a heat treatment was performed at 90°C for 3 hours, so as to give 18 g of silanized titanium oxide. A fluoride ion releasing dental composition having a composition listed in Table 2 was prepared in the same manner as in Embodiment 7 except that this silanized titanium oxide was used instead of the inorganic powder (a1), and the average fluoride releasing amount and the average calcium eluting amount of this fluoride ion releasing dental composition were obtained. The results are shown in Table 2.

### (Comparative Example 3)

A solution was obtained by dissolving 10 g of 3-methacryloyloxypropyltrimethoxysilane in 10 g of toluene, and 10 g of a titanium oxide powder was added thereto and the resultant solution was stirred. Then, the solution was distilled to take away toluene under reduced pressure, and subsequently, a heat treatment was performed at 90°C for 3 hours, so as to give 18 g of silanized titanium oxide. A fluoride ion releasing dental composition having a composition listed in Table 2 was prepared in the same manner as in Embodiment 8 except that this silanized titanium oxide was used instead of NaF, and the average fluoride releasing amount and the average calcium eluting amount of this fluoride ion releasing dental composition were obtained. The results are shown in Table 2.

### (Comparative Example 4)

A fluoride ion releasing dental composition having a composition listed in Table 2 was prepared in the same manner as in Comparative Example 1 except that fluoroaluminosilicate glass with an average particle diameter of 1 µm was used instead of 40PMF, and the average fluoride ion releasing amount and the average calcium ion eluting amount of this fluoride ion releasing dental composition were obtained. The results are shown in Table 2.

### (Comparative Example 5)

A fluoride ion releasing dental composition having a composition listed in Table 2 was prepared in the same manner as in Embodiment 2 except that the inorganic powder (a5) and fluoroaluminosilicate glass with an average particle diameter of 1 µm were used instead of the inorganic powder (a1) and 40PMF, and the average fluoride ion releasing amount and the average calcium ion eluting amount of this fluoride ion releasing dental composition were obtained. The results are shown in Table 2.

**Table 2:**

| Fluoride ion releasing dental composition | Emb. 1 | Emb .2 | Emb. 3 | Emb. 4 | Emb. 5 | Emb. 6 | Emb. 7 | Emb. 8 | Com. Ex.1 | Com. Ex.2 | Com. Ex.3 | Com. Ex.4 | Com. Ex.5 |
|---|---|---|---|---|---|---|---|---|---|---|---|---|---|
| Inorganic powder (a): | | | | | | | | | | | | | |
| Inorganic powder (a1) (wt%) | 15 | 3 | 0.5 | - | - | - | 1.9 | - | - | - | - | - | - |
| Inorganic powder (a2) (wt%) | - | - | - | 3 | - | - | - | - | - | - | - | - | - |
| Inorganic powder (a3) (wt%) | - | - | - | - | 3 | - | - | - | - | - | - | - | - |
| Inorganic powder (a4) (wt%) | - | - | - | - | - | 3 | - | - | - | - | - | - | - |
| Inorganic powder (a5) (wt%) | - | - | - | - | - | - | - | 1.9 | - | - | 1.9 | - | 3 |

| Fluoride ion releasing material: | | | | | | | | | | | | | |
|---|---|---|---|---|---|---|---|---|---|---|---|---|---|
| 40PMF (wt%) (b) | 10 | 10 | 10 | 10 | 10 | 10 | - | - | 10 | - | - | - | - |
| NaF (wt%) (b) | - | - | - | - | - | - | 1 | 1 | - | 1 | - | - | - |
| Fluoroaluminosilicate (wt%) | | | | | | | | | | | | 10 | 10 |

| Monomer (c): | | | | | | | | | | | | | |
|---|---|---|---|---|---|---|---|---|---|---|---|---|---|
| 3G (wt%) | 45 | 57 | 59.5 | 57 | 57 | 57 | 20 | 20 | 57 | 20 | 20 | 57 | 57 |
| Bis·GMA (wt%) | 28.5 | 28.5 | 28.5 | 28.5 | 28.5 | 28.5 | 40.6 | 40.6 | 28.5 | 40.6 | 40.6 | 28.5 | 28.5 |
| HEMA (wt%) | - | - | - | - | - | - | 25 | 25 | - | 25 | 25 | - | - |
| MDP (wt%) | - | - | - | - | - | - | 10 | 10 | - | 10 | 10 | - | - |

| Polymerization initiator (d): | | | | | | | | | | | | | |
|---|---|---|---|---|---|---|---|---|---|---|---|---|---|
| CQ (wt%) | 0.5 | 0.5 | 0.5 | 0.5 | 0.5 | 0.5 | 0.5 | 0.5 | 0.5 | 0.5 | 0.5 | 0.5 | 0.5 |
| DMABE (wt%) | 1 | 1 | 1 | 1 | 1 | 1 | 1 | 1 | 1 | 1 | 1 | 1 | 1 |
| Silanized titanium oxide (wt%) | - | - | - | - | - | - | - | - | 3 | 1.9 | 1 | 3 | - |
| Average fluoride ion releasing amount: 1 - 30 days | 4.2 | 5.6 | 6.3 | 4.5 | 4.1 | 8.7 | 7.5 | 25.0 | 14.2 | 17.8 | 27.3 | 6.2 | 32.6 |
| Average fluoride ion releasing amount: 31 - 60 days | 4.3 | 5.8 | 6.1 | 4.3 | 4.3 | 8.5 | 7.5 | 8.3 | 7.9 | 3.5 | 6.5 | 3.4 | 7.5 |
| Average fluoride ion releasing amount: 61 - 120 days | 4.4 | 5.8 | 6.0 | 4.6 | 3.9 | 8.3 | 7.3 | 8.0 | 5.3 | 1.5 | 2.9 | 2.2 | 2.5 |
| Average fluoride ion releasing amount: 121 - 240 days | 4.4 | 5.6 | 6.0 | 4.5 | 4.0 | 8.3 | 7.2 | 8.1 | 3.6 | 0.7 | 1.4 | 1.5 | 2.6 |
| Average fluoride ion releasing amount: 241 - 360 days | 4.3 | 5.6 | 5.7 | 4.5 | 4.2 | 8.1 | 7.3 | 7.9 | 2.1 | 0.5 | 1.2 | 0.9 | 1.9 |
| Average calcium ion eluting amount | 28 | 26 | 27 | 31 | 33 | 39 | 25 | 22 | 60 | 74 | 52 | 58 | 45 |

### (Embodiments 9 through 13)

Five kinds of fluoride ion releasing dental compositions respectively having compositions listed in Table 3 were prepared, and the average fluoride ion releasing amounts and the average calcium ion eluting amounts thereof were respectively obtained. The results are listed in Table 3.

**Table 3:**

| Fluoride ion releasing dental composition | Emb. 9 | Emb. 10 | Emb. 11 | Emb. 12 | Emb. 13 |
|---|---|---|---|---|---|
| Inorganic powder (a): | | | | | |
| Inorganic powder (a6) (wt%) | 3 | - | - | - | - |
| Inorganic powder (a7) (wt%) | - | 3 | - | - | - |
| Inorganic powder (a8) (wt%) | - | - | 3 | - | - |
| Inorganic powder (a9) (wt%) | - | - | - | 3 | - |
| Inorganic powder (a10) (wt%) | | - | - | - | 3 |

| Fluoride ion releasing material: | | | | | |
|---|---|---|---|---|---|
| 40PMF (wt%) (b) | 10 | 10 | 10 | 10 | 10 |

| Monomer (c): | | | | | |
|---|---|---|---|---|---|
| 3G (wt%) | 57 | 57 | 57 | 57 | 57 |
| Bis-GMA (wt%) | 28.5 | 28.5 | 28.5 | 28.5 | 28.5 |

| Polymerization initiator (d): | | | | | |
|---|---|---|---|---|---|
| CQ (wt%) | 0.5 | 0.5 | 0.5 | 0.5 | 0.5 |
| DMABE (wt%) | 1 | 1 | 1 | 1 | 1 |
| Average fluoride ion releasing amount: 1 - 30 days | 5.3 | 4.1 | 3.5 | 6.2 | 6.8 |
| Average fluoride ion releasing amount: 31 - 60 days | 5.6 | 4.3 | 3.4 | 6.7 | 6.5 |
| Average fluoride ion releasing amount: 61 - 120 days | 5.6 | 4.2 | 3.5 | 6.5 | 6.6 |
| Average fluoride ion releasing amount: 121 - 240 days | 5.5 | 4.3 | 3.6 | 6.5 | 6.7 |
| Average fluoride ion releasing amount: 241 - 360 days | 5.5 | 4.4 | 3.4 | 6.6 | 6.7 |
| Average calcium ion eluting amount | 22 | 29 | 32 | 27 | 29 |

As shown in Tables 2 and 3, in each of the fluoride ion releasing dental compositions prepared in Embodiments 1 through 7 and 9 through 13, the fluoride ion releasing amounts are comparatively uniform in respective periods, and the calcium ion eluting amount is small. It is understood from this fact that the fluoride ion releasing dental composition of this invention has a high fluoride ion sustained-release property and provides high acid resistance to enamel over a long period of time. Also, in the fluoride ion releasing dental composition prepared in Embodiment 8 (not within the claimed invention), the average fluoride ion releasing amount at the beginning (the period of 1 through 30 days after) is larger than in the fluoride ion releasing dental compositions prepared in the other embodiments. This is because substrate particles of the inorganic powder (a) are made of a fluoride ion releasing inorganic material.

On the other hand, the fluoride ion releasing amount is more largely reduced with time and the calcium ion eluting amount is larger in the fluoride ion releasing dental composition prepared in Comparative Example 1 than in the fluoride ion releasing dental composition prepared in Embodiment 2 and in the fluoride ion releasing dental composition prepared in Comparative Example 2 than in the fluoride ion releasing dental composition prepared in Embodiment 7. It is understood from this fact that a silane coupling agent does not have a function to improve the fluoride ion sustained-release property differently from polysiloxane. In the fluoride ion releasing dental composition prepared in Comparative Example 3, the fluoride ion releasing amounts are larger in the respective periods than in the fluoride ion releasing dental composition prepared in Comparative Example 2. It is understood from this fact that the fluoride ion releasing amounts in the respective periods can be increased by microencapsulating a fluoride ion releasing material by coating it with a polysiloxane layer. In the fluoride ion releasing dental composition prepared in Comparative Example 4, the fluoride ion releasing amounts are small in all the periods because the fluoroaluminosilicate glass has low ability to release fluoride ions. Also, in the fluoride ion releasing dental composition prepared in Comparative Example 5, the fluoride ion releasing amount is smaller and the calcium ion eluting amount is larger than in the fluoride ion releasing dental composition of the invention because the fluoroaluminosilicate glass has low ability to release fluoride ions.

## Claims

1. A fluoride ion releasing dental composition comprising:
an inorganic powder (a);
a fluoride ion releasing material (b);
a monomer (c); and
a polymerization initiator (d),
the inorganic powder (a) being made of composite particles in which a layer of polysiloxane obtained by dehydration condensing a silanol compound obtained through hydrolysis or partial hydrolysis of a silane compound represented by the following Chemical Formula 1 is formed on surfaces of substrate particles made of an inorganic material, selected from the group consisting of silicon dioxide, alumina, ceramics, diatomite, kaoline, clay mineral, activated clay, synthesized zeolite, mica, calcium phosphate, barium sulfate, titanium oxide and zirconium oxide,
the fluoride ion releasing material (b) being at least one fluoride ion releasing material selected from the group consisting of ammonium fluoride, ammonium hydrogen fluoride, a metal fluoride, a polymer including, as a constitutional unit, a vinyl monomer having an acid fluoride group and a fluorine-containing phosphazene monomer:
Chemical Formula 1: [(R¹O)ₗ(X)ₘ]₄₋ₙ-Si(-R²)ₙ
[wherein R¹ is a univalent organic group with a carbon number of 1 through 8, X is a halogen atom, R² is a univalent organic group with a carbon number of 1 through 6, one of 1 and m is 0 and the other is 1, and n is 0 or 1.]

2. The fluoride ion releasing dental composition according to Claim 1, wherein the inorganic material is titanium oxide.

3. The fluoride ion releasing dental composition according to claim 1 or 2,
wherein a weight ratio between the fluoride ion releasing material (b) and the polysiloxane is 100:0.1 through 100:1000.

4. The fluoride ion releasing dental composition according to any of Claims 1 through 3,
wherein a weight ratio between the inorganic material and the polysiloxane is 100:20 through 100:2000.

## Patentansprüche

1. Fluoridionen-freisetzende Dentalzusammensetzung, umfassend:
ein anorganisches Pulver (a),
ein Fluoridionen-freisetzendes Material (b),
ein Monomer (c) und
einen Polymerisationsinitiator (d),
wobei das anorganische Pulver (a) aus Kompositteilchen hergestellt ist, in welchen eine Polysiloxan-Schicht, erhalten durch Dehydratationskondensieren einer Silanolverbindung, erhalten durch Hydrolyse oder Teilhydrolyse einer Silanverbindung, dargestellt durch die folgende chemische Formel 1, auf Oberflächen von Substratteilchen, hergestellt aus einem anorganischen Material, ausgewählt aus der Gruppe, bestehend aus Siliciumdioxid, Aluminiumoxid, Keramiken, Diatomit, Kaolin, Tonmineral, aktiviertem Ton, synthetisiertem Zeolith, Glimmer, Calciumphosphat, Bariumsulfat, Titanoxid und Zirkoniumoxid, gebildet worden ist,
wobei das Fluoridionen-freisetzende Material (b) mindestens ein Fluoridionen-freisetzendes Material ist, ausgewählt aus der Gruppe, bestehend aus Ammoniumfluorid, Ammoniumhydrogenfluorid, einem Metallfluorid, einem Polymer einschließend als eine konstituierende Einheit ein Vinylmonomer mit einer Säurefluoridgruppe und fluorhaltiges Phosphazenmonomer:
Chemische Formel 1: [(R¹O)₁(X)ₘ]₄₋ₙ-Si(-R²)ₙ
[worin R¹ eine einwertige organische Gruppe mit einer Kohlenstoffzahl von 1 bis 8 ist, X ein Halogentatom ist, R² eine einwertige organische Gruppe mit einer Kohlenstoffzahl von 1 bis 6 ist, eines von I und m 0 ist und das andere 1 ist, und n 0 oder 1 ist.]

2. Fluoridionen-freisetzende Dentalzusammensetzung gemäß Anspruch 1, wobei das anorganische Material Titanoxid ist.

3. Fluoridionen-freisetzende Dentalzusammensetzung gemäß Anspruch 1 oder 2, wobei ein Gewichtsverhältnis zwischen dem Fluoridionen-freisetzenden Material (b) und dem Polysiloxan 100:0,1 bis 100:1000 ist.

4. Fluoridionen-freisetzende Dentalzusammensetzung gemäß Anspruch 1 oder 2, wobei ein Gewichtsverhältnis zwischen dem Fluoridionen-freisetzenden Material (b) und dem Polysiloxan 100:20 bis 100:2000 ist.

## Revendications

1. Composition dentaire libérant des ions fluor, comprenant :
une poudre inorganique (a) ;
un matériau libérant des ions fluor (b) ;
un monomère (c), et
un initiateur de polymérisation (d),
la poudre inorganique (a) étant constituée de particules composites, dans lesquelles une couche de polysiloxane obtenu par condensation par déshydratation d'un composé silanol obtenu par hydrolyse ou hydrolyse partielle d'un composé silane représenté par la formule chimique 1 suivante, est formée sur la surface de particules de substrat, composées d'un matériau inorganique, choisi parmi le groupe consistant en le dioxyde de silicium, l'alumine, des céramiques, la diatomite, le kaolin, une argile minérale, une argile activée, une zéolite synthétisée, le mica, le phosphate de calcium, le sulfate de baryum, l'oxyde de titane et l'oxyde de zirconium,
le matériau libérant des ions fluor (b) étant au moins un matériau libérant des ions fluor choisi parmi le groupe consistant en le fluorure d'ammonium, l'hydrogénofluorure d'ammonium, un fluorure métallique, un polymère comprenant comme unité constitutive, un monomère vinylique ayant un radical fluorure acide et un monomère phosphazène contenant du fluor.
Formule chimique 1 : [(R¹O)₁(X)ₘ]₄₋ₙSi(R²)ₙ
où R¹ est un radical organique monovalent ayant 1 à 8 atomes de carbone, X est un atome d'halogène, R² est un radical organique monovalent ayant 1 à 6 atomes de carbone, l'un de I et m est 0 et l'autre est 1, et n est 0 ou 1.

2. Composition dentaire libérant des ions fluor selon la revendication 1, où le matériau inorganique est l'oxyde de titane.

3. Composition dentaire libérant des ions fluor selon la revendication 1 ou 2, où le rapport pondéral entre le matériau libérant des ions fluor (b) et le polysiloxane se situe dans l'intervalle allant de 100:0,1 à 100:1000.

4. Composition dentaire libérant des ions fluor selon l'une quelconque des revendications 1 à 3, où le rapport pondéral entre le matériau inorganique et le polysiloxane se situe dans l'intervalle allant de 100:20 à 100:2000.
